# EUROPEAN PATENT APPLICATION

(11) **EP 4 365 564 A1**
(43) Date of publication of application: **08.05.2024**
(21) Application number: 22832113.9
(22) Date of filing: 29.06.2022
(51) Int. Cl.: G01M 1/38, F16K 99/00, G01N 35/10

(54) **CHIP DEVICE AND INSTRUMENT FOR NUCLEIC ACID DETECTION, AND APPLICATION THEREOF**

(30) Priority: 30.06.2021 CN 202110743068
(71) Applicant: Everlast Healthcare Limited, Kowloon, Hong Kong (CN)
(72) Inventor: ZHAO, Haifeng, Hong Kong (CN)
(74) Representative: Laine IP Oy
(86) International application number: PCT/CN2022/102391
(87) International publication number: WO 2023/274301

(57) **Abstract**

A chip device for nucleic acid detection in a sample, comprising a substrate and piston-type containers. The piston-type containers are communicated with each other by means of microfluidic channels; the chip device for nucleic acid detection is further provided with piston valves each located between containers for controlling the fluid communication between the containers communicated with each other; the chip device for nucleic acid detection comprises: a sample receiving module; a sample lysis module; a nucleic acid extraction module, optionally, the nucleic acid extraction module comprising a nucleic acid binding unit, a nucleic acid cleaning unit and a nucleic acid elution unit; and a nucleic acid amplification module. The nucleic acid amplification module comprises multiple amplification area units and an optional pre-amplification area unit. Provided is an instrument used for nucleic acid detection in a sample and using the chip device for nucleic acid detection, in particular, a POCT instrument. Provided is a method for detecting a nucleic acid in a sample by using the chip device or instrument for nucleic acid detection in a sample.

## Description

The present application claims the priority of Chinese patent application No. 202110743068.9, entitled "Chip device and instrument for nucleic acid detection, and application thereof' and filed on June 30, 2021, the entire content of which is incorporated herein by reference.

### Technical Field

The present invention relates to the field of biotechnology and application of related devices, and specifically to a chip device and instrument for detecting nucleic acids in a sample, and a method of detecting nucleic acids in a biological sample.

### Technical Background

The detection of nucleic acids is central to clinical trials, identification of pathogenic microbial types, and many other areas. Nucleic acid extraction, amplification, and assay analysis enable the detection of various diseases such as cancer, microbial infections, and genetic markers.

The use of methods such as PCR and real-time PCR is an effective method for the exponential amplification and detection of genes. The market for genetic testing using PCR/real-time PCR devices is rapidly expanding in genetic testing for infectious diseases such as viral, sexually transmitted diseases, and influenza. The role of genetic testing for cancer treatment has become evident. However, automation of applications using PCR and RT-PCR, especially small-scale automation, is not easy.

Multiplex PCR, which simultaneously amplifies multiple gene regions by using multiple pairs of primers in a single PCR system, has attracted attention. Real-time multiplex PCR, developed from multiplex PCR, is designed to detect and quantify multiple different target genes individually under conditions that are less affected (crosstalk) by other target genes and do not compromise sensitivity. However, it has been reported that overlapping wavelengths of labelable fluorescent substances and species issues make more than two quantitative multiplex reactions often difficult to perform.

Currently, two major problems with small flow channel systems include the power source and the valve problem. The power source provides the impetus for the movement of the microfluid within the carrier (e.g., chip, tube, etc.). Currently, commonly used methods include injection, centrifugal, and pneumatic pressure. Valves, on the other hand, control the motion behavior of the microfluid within the carrier. Due to the small scale of microfluidics, it is very difficult to construct valves with sufficient precision, in sufficient number and easy to open and close inside their carriers. For laboratories studying microfluids, syringe pumps are the most commonly used power source. By drawing fluid into a syringe, connecting the syringe to the inlet of a microfluidic carrier (hereafter referred to as the carrier), and then pushing the syringe with a high-precision syringe pump, the microfluidic injection into the carrier is realized. However, this operation has many steps, and it is difficult to switch the fluid samples, and it is difficult to realize the automated feeding of the microfluid.

Therefore, there is a need in this field for high-speed real-time, compact and easy-to-operate nucleic acid amplification and detection devices and instruments, especially microarray devices and instruments suitable for point-of-care testing (POCT).

### Summary of the Invention

The invention provides a chip device for detecting nucleic acid in a sample, which has a substrate and piston-style containers, said piston-style containers being in communication with each other through a microfluidic channel, wherein each piston-style container comprises a chamber (or "cavity") and a piston disposed within the chamber, the chamber having an opening in communication with said microfluidic channel (or "flow channel" in the following description) at a bottom thereof.

In one aspect of the present invention, the chip device further has a piston-style valve (or "piston valve") located between interconnected containers for controlling fluid communication between the interconnected containers, and the piston valve includes a valve chamber and a valve disposed within the chamber, the valve chamber having two or more openings at the bottom being in communication with the microfluidic channel, the valve piston being configured to move to the bottom of the chamber for covering said openings, blocking communication of the chamber with the microfluidic channel, thereby blocking fluidic communication between containers connected through said microfluidic channels, preferably, the microfluidic channel communicates with the opening at the bottom of the chamber from the bottom of the chamber through an upward flow channel.

In one aspect of the present invention, the chip device includes one or any combination or all of the following modules:
a sample receiving module;
a sample lysis and nucleic acid extraction module, used to perform a lysis reaction and/or a nucleic acid extraction reaction on the sample to obtain purified nucleic acids from the sample; said sample lysis and nucleic acid extraction module comprising a sample lysis module and/or a nucleic acid extraction module, and optionally, said nucleic acid extraction module comprising a nucleic acid binding unit, a nucleic acid washing unit, and a nucleic acid elution unit;
   and
a nucleic acid amplification module, for distributing nucleic acid samples to a plurality of amplification zone containers and for amplifying and/or detecting nucleic acid molecules, the nucleic acid amplification module comprising a multiplexed amplification zone module and, optionally, a preamplification zone module.

In one aspect of the present invention, the chip device includes the following modules:
a sample receiving module;
a sample lysis and nucleic acid extraction module, used to perform a lysis reaction and/or a nucleic acid extraction reaction on a sample to obtain purified nucleic acids from the sample; said sample lysis and nucleic acid extraction module having a sample lysis module and/or a nucleic acid extraction module, and optionally, said nucleic acid extraction module comprising a nucleic acid binding unit, a nucleic acid washing unit, and a nucleic acid elution unit;
   and
a nucleic acid amplification module, for distributing nucleic acid samples to a plurality of amplification zone containers and for amplifying and/or detecting nucleic acid molecules, the nucleic acid amplification module comprising a multiplexed amplification zone module and, optionally, a preamplification zone module.

In one aspect of the present invention, the sample-receiving module comprises a sample loading chamber, which is a piston-style container comprising a chamber and a piston disposed within the chamber. Optionally, the sample-receiving module comprises a sample receiving chamber and a sample loading chamber. Further optionally, it also comprises a sample loading chamber valve between the sample receiving chamber and the sample loading chamber, which is a piston valve, including a valve chamber and a valve disposed within the chamber, the bottom of the valve chamber having two openings communicating with the sample receiving chamber and the sample loading chamber, the valve piston can move to the bottom of the chamber and cover said openings, thereby blocking fluidic communication between the sample receiving chamber and the sample loading chamber.

In one aspect of the present invention, the sample lysis and nucleic acid extraction module comprises a first mixing chamber and a second mixing chamber, each of said first mixing chamber and said second mixing chamber being a piston-style container comprising a chamber and a piston disposed within the chamber. Optionally, a mixing chamber valve is arranged between said first mixing chamber and said second mixing chamber, for example, it is a piston valve, including a valve chamber and a valve piston located in the valve chamber, having two openings communicating with said first mixing chamber and said second mixing chamber, the valve piston can move to the bottom of the chamber and cover said openings, thereby blocking fluidic communication between said first mixing chamber and said second mixing chamber.

In another aspect of the present invention, a sampling valve is arranged between the first mixing chamber and the upstream sample receiving module (such as the sample loading chamber therein), which is, for example, a piston valve, including a valve cavity and a valve cavity located in the valve cavity. The bottom of the valve cavity has two openings respectively communicated with the sample loading chamber and the first mixing chamber, the piston can move to the bottom of the cavity and cover the opening, thus blocking fluidic communication between the sample loading chamber and the first mixing chamber.

In one aspect of the present invention, the sample lysis module of the sample lysis and nucleic acid extraction module in the chip device comprises a lysis reagent storage chamber for containing the lysis reagent, which is a piston-style container, including a cavity and a piston. Optionally, the sample lysing module also comprises a lysing reagent valve between the lysing reagent storage chamber and the chamber for sample lysing, the lysing reagent valve is, for example, a piston valve, including a valve chamber and a valve chamber located in the valve chamber. The valve piston has two openings at the bottom of the valve chamber which are connected with the lysis reagent storage chamber and the sample lysis chamber respectively. The piston can move to the bottom of the chamber and cover the openings, thus blocking the fluidic communication between the lysis reagent storage chamber and the sample lysis chamber.

In one aspect of the present invention, the sample lysis and nucleic acid extraction module in the chip device has one or more of a binding unit, a cleaning unit, and an eluting unit, which respectively have a nucleic acid binding reagent, a nucleic acid Nucleic acid binding reagent storage cavity, nucleic acid cleaning reagent storage cavity and nucleic acid elution reagent storage cavity for cleaning reagent and nucleic acid eluting reagent. The nucleic acid binding reagent storage chamber, the nucleic acid cleaning reagent storage chamber and the nucleic acid elution reagent storage chamber are piston-type containers, including a cavity body and a piston located in the cavity. Optionally, the nucleic acid binding reagent storage chamber, nucleic acid cleaning reagent storage chamber and nucleic acid eluting reagent storage chamber are each connected to a chamber in which the nucleic acid binding, nucleic acid reagent or nucleic acid eluting reaction is carried out via a nucleic acid binding reagent valve, a nucleic acid cleaning reagent valve or a nucleic acid eluting reagent valve, respectively, and each of said nucleic acid binding reagent valve, nucleic acid cleaning reagent valve or nucleic acid eluting reagent valve is a piston valve.

In one aspect of the present invention, the chamber for sample lysis and the chamber for nucleic acid binding, nucleic acid reagent and/or nucleic acid elution reaction in the chip device is the first mixing cavity and/or the second mixing cavity. mixing chamber.

In one aspect of the present invention, the nucleic acid extraction module in the chip device also includes a water adding chamber, which is connected to one or more of the lysing reagent storage chamber, the nucleic acid binding reagent storage chamber, the nucleic acid cleaning reagent storage chamber and the nucleic acid elution reagent storage chamber through a flow channel. Optionally, there is a valve between the water adding chamber and one or more of the lysing reagent storage chamber, the nucleic acid binding reagent storage chamber, the nucleic acid cleaning reagent storage chamber and the nucleic acid elution reagent storage chamber, while the valve is, for example, a piston valve.

In one aspect of the present invention, the lysing reagent storage chamber and/or the nucleic acid binding reagent storage chamber in the chip device is the first mixing chamber and/or the second mixing chamber.

In one aspect of the present invention, the sample lysis and extraction module in the chip device further includes a chamber for the sample to be amplified (or sample-to-be-amplified chamber), which is a piston-style container, including a chamber and a piston located in the chamber, for containing the lysed and extracted nucleic acid solution samples. Optionally, there is also a piston valve upstream of the sample-to-be-amplified chamber, which includes a valve cavity and a valve piston located in the valve cavity, and the bottom of the valve cavity is respectively connected to the upstream module or unit and the downstream amplification module. The valve piston has two openings at the bottom of the valve chamber which are connected with the upstream module or unit and the downstream amplification module respectively. The piston can move to the bottom of the chamber and cover the openings, thus blocking the fluidic communication between the upstream module or unit and the downstream amplification module.

In one aspect of the present invention, the sample lysis and extraction module in the chip device further comprises a dilution chamber. Optionally, a dilution valve, such as a piston-style container, is arranged between the dilution chamber and its upstream module or unit (e.g., the second mixing chamber).

In one aspect of the present invention, the sample lysis and extraction module in the chip device further comprises a filter unit. In another aspect of the present invention, the filter unit is a filter chamber, which is a piston-type container with a piston in the cavity, and two openings at the bottom of the cavity that communicate with the flow channel, and the flow channel is respectively connected to the upstream diluting chamber and downstream sample-to-be-amplified chamber, and a filter is arranged between the bottom of the chamber and the piston for filtering unwanted substances (such as cells, cell fragments or large protein molecules, etc.) in the nucleic acid extraction solution.

In one aspect of the present invention, in the chip device, the nucleic acid amplification module has one or more multiple amplification region units. The multiple amplification area unit includes a plurality of amplification reaction chambers (set in the bottom plate) for performing nucleic acid amplification reactions, and the nucleic acid sample solution from the sample lysis and extraction module is distributed to each of the amplification reaction chambers through a microfluidic channel.

In one aspect of the present invention, in the chip device, between the multiple amplification area unit and the upstream sample lysing and extraction module (such as between the multiple amplification area unit and the upstream sample-to-be-amplified chamber) has a piston valve, the valve cavity has a piston, and the bottom of the valve cavity has an opening that communicates with the flow channel that communicates with the upstream sample-to-be-amplified chamber, and an opening that communicates with the downstream amplification region unit.

In one aspect of the present invention, in the chip device, the multiple amplification reaction chambers of the multiple amplification zone unit are distributed along a circumferential direction. Optionally, a multiple amplification sampling valve is arranged thereabove, which is a piston valve, with a piston in the valve cavity, and with an opening at the bottom of the valve cavity that communicates with the flow channel of the upstream sample cavity to be amplified, and with a plurality of openings respectively communicating with each of the plurality of amplification reaction chambers therebelow.

In one aspect of the present invention, in the chip device, the multiple amplification reaction chambers of the multiple amplification zone unit is arranged in an array. Optionally, each amplification reaction chamber communicates with the main channel of the amplification zone unit through a branch flow channel, and the main channel of the multiple amplification zone unit communicates with the upstream sample lysis and extraction module.

In one aspect of the present invention, in the chip device, there is a piston valve between the multiple amplification region unit and the upstream sample lysing and extraction module, a piston in the valve cavity, and a valve at the bottom of the valve cavity, with the opening communicates with the flow channel of the upstream sample-to-be-amplified chamber, and the opening communicates with the main channel of the downstream amplification zone unit.

In one aspect of the present invention, in the chip device, the nucleic acid amplification module has an inert liquid module for storing and providing an inert liquid (such as mineral oil or paraffin oil) for covering an interface between the amplification reaction chamber and the flow channel. In yet another aspect of the present invention, the inert liquid module has an oil filling chamber. Optionally, the inert liquid module also includes a oil filling valve between the oil filling chamber and the multiple amplification zone unit, which is, for example, a piston valve, with a piston in the valve chamber, and a flow channel connected to the oil filling chamber at the bottom of the valve chamber with an opening communicating with the channel, and an opening communicating with the amplification region unit.

In one aspect of the present invention, in the chip device, the nucleic acid amplification module further has a pre-amplification area unit arranged upstream of the multiple amplification area unit for performing the first round of amplification on nucleic acid molecules, such as nested amplification. In still another aspect of the present invention, the pre-amplification unit has a nested amplification chamber, for example, it is a cylindrical cavity arranged in the bottom plate, which has flow channels communicating with a upstream sample lysing and extraction module and a downstream reamplification zone unit.

In one aspect of the present invention, in the chip device, the amplification reaction chambers of the amplification reaction unit are isolated by materials that completely absorb or substantially completely absorb signals generated by the amplification reaction.

In one aspect of the present invention, in the chip device, the bottom of each amplification reaction chamber of the amplification reaction unit is prepared or sealed with a material that does not absorb at all or substantially does not absorb signals generated by the amplification reaction.

In one aspect of the present invention, in the chip device, the piston valve has a piston movement control member disposed above the valve piston, for controlling the up and down movement of the valve piston in the cavity.

In one aspect of the present invention, in the chip device, the chamber wall of the valve chamber of the piston valve has an internal thread.

In one aspect of the present invention, in the chip device, the piston movement control member has an external thread, which forms a thread pair with the internal thread of the chamber wall, and the piston motion control member rotates along the thread to move within the chamber for driving the piston to move in the chamber.

In one aspect of the present invention, in the chip device, the piston movement control member has a cavity suitable for receiving a control rod, and the cross section of the cavity is adapted to the control rod.

In one aspect of the present invention, in the chip device, the bottom of the valve piston of the piston valve mechanism has a piston support body, and the piston support body is a protrusion at the bottom of the piston, which is made of elastic material.

In one aspect of the present invention, in the chip device, the cross-sectional diameter of the piston container or piston valve is about 0.5mm-25mm, preferably about 1-20mm, more preferably about 3-15mm.

The present invention also provides an instrument for detecting nucleic acid in a sample, especially a POCT instrument, which includes the aforementioned chip device provided by the present invention. The chip device has a substrate and piston-style containers, said piston-style containers communicates with each other through a microfluidic channel, wherein said piston-style container comprises a chamber and a piston disposed within the chamber, the bottom of the chamber having an opening communicating with said microfluidic channel,
said chip device further has a piston valve disposed between the containers for controlling fluidic communication between the interconnected containers, said piston valve comprising a valve chamber and a valve piston disposed within the valve chamber, the bottom of the valve chamber having two or more openings communicating with the flow channel, the valve piston can move to the bottom of the chamber and cover said openings, blocking communication of the chamber with the fluid channel, thereby blocking fluidic communication between containers connected through said fluid channels,
wherein said chip device comprises:
   a sample receiving module;
   a sample lysis and nucleic acid extraction module, which may be used to perform a lysis reaction and/or a nucleic acid extraction reaction on a sample to obtain purified nucleic acids from the sample; said sample lysis and nucleic acid extraction module having a sample lysis module and/or a nucleic acid extraction module, and optionally, said nucleic acid extraction module comprising a nucleic acid binding unit, a nucleic acid washing unit, and a nucleic acid elution unit;
      and
   a nucleic acid amplification module for distributing nucleic acid samples to a plurality of amplification zone containers and for amplifying and/or detecting nucleic acid molecules; the nucleic acid amplification module comprising a multiplexed amplification zone module and, optionally, a preamplification zone module.

In one aspect of the present invention, the instrument has a chip device receiving and movement control system for receiving the above chip device and transferring it to a designated position in the instrument to perform various processes on the chip.

In one aspect of the present invention, the instrument has a magnet and a system for controlling the movement of the magnet, and is used for purifying nucleic acid in a sample using a magnetic bead method. In the magnetic bead method, the nucleic acid is bound by contacting the nucleic acid-binding magnetic material and/or binding solution with the sample that has been lysed, thereby allowing the nucleic acid to bind to the magnetic material. The complex formed after the nucleic acid is combined with the magnetic material can controllably move, stir or precipitate in the container under the action of a magnetic field, so as to achieve the purposes of nucleic acid binding, nucleic acid cleaning and nucleic acid elution.

In one aspect of the invention, the instrument has a control mechanism for regulating the movement of a piston motion control in the piston valve. In one aspect of the present invention, the control mechanism includes a control rod and a control rod movement mechanism, the control rod can rotate or move up and down, wherein the control rod movement mechanism includes a component for controlling the control rod up and down movement and rotation, Such as motors.

In one aspect of the present invention, the instrument has a signal detection module for detecting nucleic acid amplification products, such as a fluorescence detection system.

In one aspect of the present invention, the instrument has a temperature control system for the nucleic acid amplification region of the chip.

In one aspect of the present invention, the instrument has a nucleic acid amplification result analysis and/or output system.

In one aspect of the present invention, a method for detecting nucleic acid in a sample using the chip device or instrument of the present invention is provided.

In one aspect of the present invention, the detection method comprises the following steps:
provide the above-mentioned chip device or an instrument having the chip device, said chip device having a substrate and piston-style containers, said piston-style containers being connected to each other by a microfluidic channel, wherein said piston-style container comprises a chamber and a piston disposed within the chamber, the bottom of the chamber having an opening communicating with said microfluidic channel,
said chip device further has a piston valve disposed between the containers for controlling fluidic communication between the interconnected containers, said piston valve comprising a valve chamber and a valve piston disposed within the valve chamber, the bottom of the valve chamber having two or more openings communicating with the flow channel, the valve piston can move to the bottom of the chamber and cover said openings, blocking communication of the chamber with the fluid channel, thereby blocking fluidic communication between containers connected through said fluid channels (the fluid channel communicates with the opening at the bottom of the chamber from the bottom of the cavity through an upward flow channel).

As mentioned above, the chip device may include:
a sample receiving module;
a sample lysis and nucleic acid extraction module, which may be used to perform a lysis reaction and/or a nucleic acid extraction reaction on a sample to obtain purified nucleic acids from the sample; said sample lysis and nucleic acid extraction module having a sample lysis module and/or a nucleic acid extraction module, and optionally, said nucleic acid extraction module comprising a nucleic acid binding unit, a nucleic acid washing unit, and a nucleic acid elution unit;
   and
a nucleic acid amplification module for dispensing nucleic acid samples to a plurality of amplification zone containers and for amplifying and/or detecting nucleic acid molecules; the nucleic acid amplification module comprising a multiplexed amplification zone module and, optionally, a preamplification zone module.

As previously mentioned, the apparatus optionally includes:
a chip device receiving and motion control system;
magnets and a system for controlling the movement of the magnets;
a control mechanism for regulating the movement of a piston motion control member in said piston valve, said control mechanism comprising a control rod and a control rod motion mechanism, the control rod being capable of rotational movement or up and down movement, wherein said control rod motion mechanism comprises a component, such as a motor, for controlling up and down or rotational movement of said control rod;
a signal detection module for detecting nucleic acid amplification products, for example a fluorescence detection system;
a system for temperature control of the nucleic acid amplification region of said chip;
a system for analyzing and/or outputting nucleic acid amplification results.

In one aspect of the present invention, the method optionally includes the steps of:
adding the sample to be tested through the sample receiving module of the chip device;
performing a lysis reaction and/or nucleic acid extraction reaction on the sample through the sample lysis and nucleic acid extraction module of the chip device, and obtaining purified nucleic acid from the sample;
the nucleic acid amplification module is used for distributing nucleic acid samples to multiple amplification zone containers and performing amplification and/or detection on nucleic acid molecules.

In one aspect of the present invention, the diagnostic instrument is used for identification of infection source, genetic disease, cancer detection or gene variation detection.

In one aspect of the present invention, the diagnostic instrument is used for the detection of the following pathogens: coronavirus, influenza virus, enterovirus, hepatitis B virus, hepatitis C virus, Ebola virus, Marburg virus, SARS virus, Zika virus , Bunia virus, rhinovirus, respiratory nucleocytosis virus, cholera virus and other viral pathogens, or mycobacterium tuberculosis, Escherichia coli, Acinetobacter baumannii, Diplococcus pneumoniae, Streptococcus lactis, Sarcina urea, Staphylococcus aureus, Bacillus subtilis, Bacillus anthracis, Bacillus subtilis, Streptococcus, Proteus, Vibrio cholerae, Treponema pallidum and other bacterial pathogens.

In one aspect of the present invention, the diagnostic instrument is used for the detection of the following cancers: leukemia, Hodgkin's disease, Wilm's tumor (Wilms tumor), melanoma, retinoblastoma, gastric cancer, liver cancer, lung cancer , Esophageal cancer, cervical cancer, breast cancer, colon cancer, rectal cancer, nasopharyngeal cancer, ovarian cancer, kidney cancer, bladder cancer, thyroid cancer, skin cancer, etc.

### Description of Drawings

In order to more clearly illustrate the technical solutions in the embodiments of the present invention or the prior art, the following will briefly introduce the drawings that need to be used in the description of the embodiments or the prior art. Obviously, the accompanying drawings in the following description These are some embodiments of the present invention. For those skilled in the art, other drawings can also be obtained according to these drawings without any creative effort.
FIG. 1 is a stereoscopic perspective view of an exemplary chip device provided by the present invention for nucleic acid detection in a sample.
FIG. 2 is another perspective view of the exemplary chip device for nucleic acid detection in a sample shown in FIG. 1. Compared with FIG. 1, FIG. 2 does not show the internal thread on the chamber wall of some piston-style containers or piston valves in the chip device and the piston movement control bodies in the chamber of the piston valve.
FIG. 3 is a schematic top plan view of the structure of the exemplary chip device for detecting nucleic acid in a sample shown in FIG. 1 and the communication relationship of its flow channels.
FIG. 4 is a schematic diagram of the structure and working mode of an exemplary piston valve and piston motion control mechanism in the chip device for nucleic acid detection in samples provided by the present invention. FIG. 4 (a) is a schematic cross-sectional view of the structure of the exemplary piston valve. FIG 4 (b) and (c) are schematic diagrams of the working mode of the exemplary piston valve. FIG. 4 (b) shows an exemplary working mode of controlling the downward movement of the piston, and FIG. 4(c) shows an exemplary working mode of controlling the upward movement of the piston.
FIG. 5 is a perspective view of yet another exemplary chip device used in the detection of nucleic acid in a sample provided by the present invention. The figure does not show the internal thread on the cavity wall of some piston containers or piston valves in the chip device provided by the present invention and the piston movement control parts in the cavity of the piston valve.
FIG. 6 is another perspective view of the exemplary chip device for nucleic acid detection in a sample shown in FIG. 5. The figure does not show the piston in the piston container or the piston valve in the chip device provided by the present invention.
FIG. 7 is a schematic diagram of the top-view structure of the exemplary chip device for detecting nucleic acid in a sample shown in FIG. 5 and the communication relationship of its flow channels.

### Detailed Description

In order to make the purpose, technical solutions and advantages of the embodiments of the present invention clearer, the technical solutions in the embodiments of the present invention will be clearly and completely described below in conjunction with the drawings in the embodiments of the present invention. Obviously, the described embodiments are a part of embodiments of the present invention, but not all embodiments. Based on the embodiments of the present invention, all other embodiments obtained by persons of ordinary skill in the art without creative efforts belong to the scope of protection of the present invention.

### Example 1

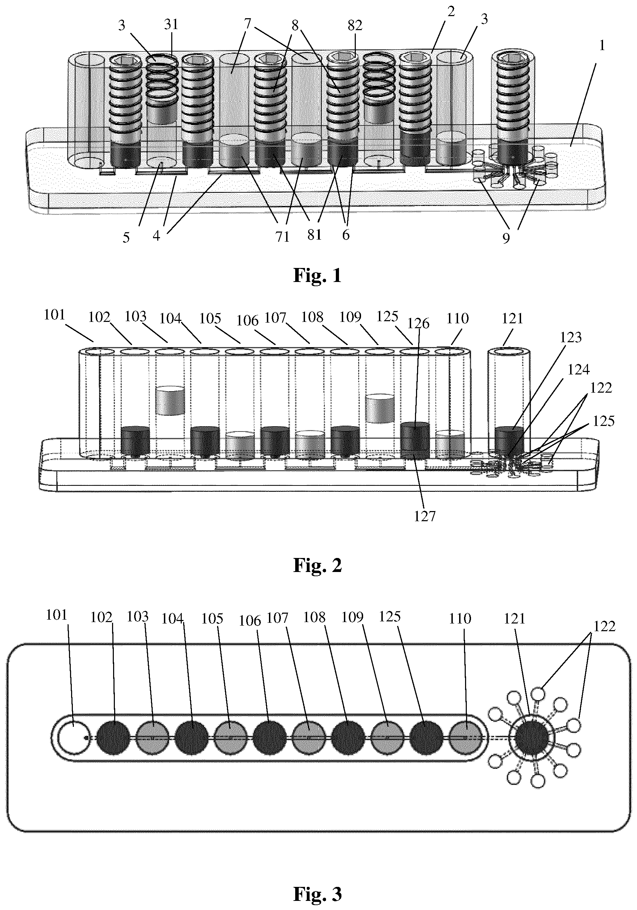

The invention provides a chip device for nucleic acid detection in samples. The chip device is usually used to extract and amplify nucleic acid in a sample and detect its presence and amount. Fig. 1 is a stereoscopic perspective view of an exemplary chip device provided by the present invention for nucleic acid detection in a sample. As shown in Figure 1, the chip device provided by the present invention has a substrate 1 and a container area 2 perpendicular to the substrate, columnar containers 3 arranged in parallel is arranged in the container area, and a micro flow channel 4 is arranged in the substrate below the container area, the bottom of chamber (or named "cavity") of the columnar container has an opening 5 communicating with the microfluidic channel, and fluidic communication between the columnar containers can be realized through the microfluidic channel. There is a piston 6 movable in the chamber in the columnar container, and the piston and the chamber constitute a piston-style container (or "piston container" in short, such as the columnar piston-style container 7 in Fig. 1). Piston containers are used to contain a sample or formulation (solid or liquid formulation), which resides in the space between the piston and the bottom of the chamber. The bottom of the chamber has an opening communicating with the fluid channel, and the solution is transferred between the containers through the micro flow channel. The columnar container also includes a piston-style valve (or named "piston valve" in the specification, such as the piston valve 8 in Fig. 1) for controlling fluidic communication between the interconnected containers. there are two or more openings at the bottom of the valve chamber communicating with the flow channel, the valve piston can move to the bottom of the chamber and cover said openings, blocking communication of the chamber with the fluid channel, thereby blocking fluidic communication between upstream and downstream containers connected through said fluid channels. Preferably, in the chip device provided by the present invention, the microfluidic channel communicates with the opening at the bottom of the cavity from below the cavity of the piston container or piston valve through an upward channel. The chip device provided by the present invention is usually used for temperature-controlled amplification reaction of nucleic acid, and the temperature-controlled amplification reaction is usually carried out in multiple reaction chambers 9 arranged in the substrate.

The chip device provided by the invention is used for detecting the presence and quantity of nucleic acid in a sample after extraction and amplification. The chip device provided by the present invention may include one or more of the following modules:
a sample receiving module for receiving, dispensing, and holding an appropriate amount of sample for testing;
a sample lysis and nucleic acid extraction module, this module is used to accommodate lysis and nucleic acid extraction reagents, perform lysis reaction and/or nucleic acid extraction reaction on the sample, and obtain purified nucleic acid from the sample for the next step of nucleic acid amplification reaction. Correspondingly, the sample lysis and extraction module may include a sample lysis module for accommodating lysis reagents and performing a lysis reaction on the sample, and may include a nucleic acid extraction module that may include a nucleic acid binding unit, a nucleic acid cleaning unit, and a nucleic acid elution unit , which are respectively used to accommodate nucleic acid binding reagents, nucleic acid cleaning reagents and nucleic acid elution reagents, and perform reactions such as nucleic acid binding, nucleic acid reagents, and nucleic acid elution respectively;
a nucleic acid amplification module, which is used for distributing the nucleic acid extracted from the sample to the nucleic acid amplification reaction chamber and amplifying the nucleic acid molecule, especially performing nucleic acid multiple amplifications in multiple nucleic acid amplification reaction chambers. Optionally, a first round of nucleic acid amplification can also be performed prior to the multiple nucleic acid amplification, and the first round of nucleic acid amplification may be, for example, a nested amplification. Correspondingly, the nucleic acid amplification module may include a multiple amplification region module and an optional pre-amplification region module.

The containers used to hold solid or liquid preparations or carry out reacts in each module of the chip device provided by the present invention can be piston-style containers, and the interconnected containers are connected through fluid channels arranged in the lower substrate. The piston-type container includes a chamber and a piston located in the chamber, the bottom of the chamber has an opening communicating with the fluid channel, the solid or liquid preparation can be hold in the space between the piston and the bottom of the chamber, and the solution can pass through the microchannel in the transfer between containers.

In the chip device provided by the present invention, there can be piston valves provided between the interconnected containers. The piston valve includes a valve chamber and a valve piston located in the valve chamber, and the bottom of the valve chamber has two or more openings communicating with the flow channel. The fluid channel communicates with the opening at the bottom of the chamber from below the chamber through an upward flow channel. In one aspect of the present invention, the opening is not located on the side wall of the chamber of the piston valve. A piston is movable to the bottom of the chamber and covers the opening, blocking communication of the chamber with the fluid channel, thereby blocking fluidic communication between containers connected by the fluid channel.

The chip devices described herein are applicable to the analysis of any nucleic acid-containing sample for any purpose, including but not limited to genetic testing for human genes and clinical testing for various infectious diseases. Nucleic acid samples for use in the methods described herein may be from any source. Typically, a sample may be biological material isolated from its natural environment and comprising polynucleotides. A sample may consist of a purified or isolated polynucleotide, or may comprise a biological sample such as a tissue sample, a biological fluid sample or a cell sample comprising the polynucleotide. Biological fluids include blood, plasma, sputum, urine, cerebrospinal fluid, lavage fluid samples as non-limiting examples. Nucleic acid samples may be of plant, animal, bacterial or viral origin. Samples may be obtained from various sources, including but not limited to, from different individuals, different developmental stages of the same or different individuals, different diseased individuals, normal individuals, different disease stages of the same or different individuals, individuals undergoing different disease treatments, in samples of individuals with different environmental factors, or individuals with predisposition to disease, or individuals exposed to infectious disease agents.

The term "unit" as used herein is intended to denote an element or combination of elements configured to operate together to perform one or more functions or produce one or more desired results, wherein each element may have a distinct and/or independent functions. It will be understood that each element within a unit need not be directly connected to each other element.

The term "module" used herein is used to denote a unit or a combination of units configured to operate together to realize the functions of one or more subsystems of the device of the present invention. It should be understood that each unit within a module need not be directly connected to each other unit.

In order to better understand and explain the invention, the invention will be further described in detail below with reference to the accompanying drawings.

Fig. 1 is a stereoscopic perspective view of an exemplary chip device provided by the present invention for nucleic acid detection in a sample. FIG. 2 is another perspective view of the exemplary chip device for nucleic acid detection in a sample shown in FIG. 1. Compared with FIG. 1, FIG. 2 does not show the internal thread on the chamber wall of some piston-style containers or piston valves in the chip device and the piston movement control bodies in the chamber of the piston valve. FIG. 3 is a schematic top plan view of the structure of the exemplary chip device for detecting nucleic acid in a sample shown in FIG. 1 and the communication relationship of its flow channels.

As shown in FIG. 1 and FIG. 2, the chip device for detecting nucleic acid in a sample provided by the present invention includes a substrate 1 and a container area 2 perpendicular to the substrate, and a plurality of cylindrical containers 3 are arranged in the container area 2. The cylindrical chamber is formed with wall of the container area perpendicular to the bottom plate, and the bottom of the chamber has an opening 5 communicating with a fluid channel 4 provided in the bottom plate. The cylindrical chambers communicate with each other through fluid channels 4 provided in the bottom plate. The fluid channel is located below the chamber, and communicates with the opening at the bottom of the cavity through an upward flow channel.

The substrate and container region are made of rigid material. Such materials include, but are not limited to, silica, silicon, quartz, glass, or polymeric materials (e.g., PDMS, plastic, etc.). The fluid channel provided in the substrate is generally a microfluidic channel, the size of which is on the order of millimeters, for example, the width of the cross-section of the channel is about 0.1-5 mm. In the present invention, the terms "fluidic channel", "microfluidic channel" and "microfluidic passage" are used interchangeably. The cross section of the channel of the fluidic channel can be in various shapes, including ellipse, rectangle, square, circle and so on. In one aspect of the present invention, the cross-section of the flow channel has a width of about 0.1-5 mm, preferably about 0.2 mm-2 mm.

In one aspect of the present invention, since the nucleic acid is detected by detecting the fluorescent signal carried by the amplified nucleic acid. In order to avoid or eliminate signal interference caused by the adjacent nucleic acid amplification regions in the nucleic acid amplification module, the wall of the cylindrical chamber and the bottom plate can be made of materials that completely absorb or substantially absorb the target signal (such as fluorescence). The bottom of the amplification reaction area (usually a cavity) is prepared or sealed with a material that does not absorb at all or substantially does not absorb the signal to be detected. Thus, the signal detection module disposed in the chip or an external detection system can detect the fluorescent signals generated in each amplification reaction region through the bottom of the amplification reaction region.

In the chip device of the present invention as shown in FIG. 1, the plurality of cylindrical chambers include a piston-style container, such as the piston-style container 7 in FIG 1, which consists of a chamber surrounded by a chamber wall perpendicular to the bottom plate and a piston 71 in the chamber, the piston can move up and down in the chamber. The plurality of piston-style containers communicate with each other through fluid channels 4 provided in the base plate. The fluid channel is located below the chamber and communicates with the opening 5 at the bottom of the chamber through an upward flow channel. In the device of the present invention as shown in FIG. 1, piston valves are also included in the plurality of cylindrical chambers, such as piston valve 8 in FIG. 1, for controlling the fluidic communication between the interconnected piston containers. The piston valve consists of a valve chamber surrounded by a wall perpendicular to the bottom plate and a valve piston 81 located in the valve chamber, and the bottom of the valve chamber (not the side wall) has two or more openings 6 in communication with flow passages connected to the upstream and downstream containers controlled by it respectively. The valve piston 81 can move to the bottom of the chamber and cover the opening 6, blocking the communication between the chamber and the fluid channel, thereby blocking the connection between the containers connected by the fluid channel. The fluid channel communicates with the opening at the bottom of the valve chamber through an upward channel from the bottom of the valve chamber.

FIG. 2 is another perspective view of the partial structure of the exemplary chip device for detecting nucleic acid in a sample provided by the present invention in FIG. 1. Compared with FIG. 1, FIG. 2 does not show the internal thread on the chamber wall of the piston valve used in the chip device provided by the present invention and the piston movement control member in the chamber. As shown in FIG. 2, the device for nucleic acid detection in a sample provided by the present invention has a sample receiving module, including a sample receiving chamber 101 and a sample loading chamber 103, and a sampling chamber valve 102 located between the sample receiving chamber 101 and the sample loading chamber 103. The original sample to be tested can be added into the sample receiving chamber 101. When the concentration of the original sample to be tested is greater than the designed concentration, water or other suitable solutions such as PBS buffer can be added to the sample receiving chamber. The sampling chamber valve 102 is a piston valve, with a piston in the valve chamber and two openings at the bottom of the valve chamber communicating with the flow channels in the substrate, which communicating with the sample receiving chamber 101 and the sample loading chamber 103 respectively. The sample loading chamber 103 is a piston-type container with a piston in the chamber and an opening at the bottom of the chamber that communicates with the flow channel in the substrate. In an exemplary working situation, the initial state of the device for nucleic acid detection in a sample is: the sampling chamber valve 102 is in a closed state (that is, the piston of the sampling chamber valve 102 is located at the bottom of the chamber and covers the opening, blocking the communication between the chamber and the fluid channel), the piston in the sample loading chamber 103 is located at a specified height (at this time, the volume between the piston and the bottom of the sample loading chamber is the designed volume, for example, the designed sample volume for the sample extraction reaction). When keeping the sampling chamber valve 102 in the closed state, add the sample in the sample receiving chamber 101; then the sampling chamber valve 102 is opened (the piston of the sampling chamber valve 102 is raised), because the initial state of the sample loading chamber 103 is a vacuum state, the solution in the sample receiving chamber 101 of the specified volume is sucked into the sample loading chamber 103 through the sampling chamber valve 102; the sampling chamber valve 102 is then closed (the piston of the sampling chamber valve 102 is pushed to the bottom of the chamber).

As shown in FIG. 2, the chip device used for nucleic acid detection in samples provided by the present invention has a sample lysis and nucleic acid extraction module, which includes a first mixing chamber 105 and a second mixing chamber 107, and there is a mixing chamber valve 106 between said first mixing chamber 105 and said second mixing chamber 107, which is a piston valve. There is also a sampling valve 104 between the sample loading chamber 103 and the first mixing chamber 105. Wherein, the first mixing chamber 105 and the second mixing chamber 107 are both piston-type containers, with a piston inside the chamber, and an opening at the bottom of the chamber communicating with the flow channel in the substrate. The sampling valve 104 and the mixing chamber valve 106 are both piston valves, with a piston in the valve chamber, and two openings at the bottom of the valve chamber that communicate with the flow channel in the substrate, and the two openings are respectively connected with the flow channels of upstream and downstream containers: the two flow channels controlled by the sampling valve 104 communicate with the first mixing chamber 105 and the sample loading chamber 103 respectively, and the two flow channels controlled by the mixing chamber valve 106 communicate with the first mixing chamber 105 and the second mixing chamber 107 respectively.

The chip device for nucleic acid detection in this embodiment is particularly suitable for nucleic acid extraction from a sample by direct extraction, that is, the solution obtained after the sample contacts and reacts with a nucleic acid extraction reagent contains nucleic acid that can be used for subsequent amplification reactions. Samples suitable for nucleic acid extraction and amplification by direct extraction include artificially synthesized cloning liquid, in vitro transcribed RNA, plasmids, serum, plasma, urine, cotton swab eluate, sputum, alveolar lavage fluid, etc. The nucleic acid extraction reagents used to extract the above samples usually contain lysing agents, including various surfactants such as SDS, Triton, NP-40, etc., and other chemical reagents such as buffers, protease inhibitors, reducing agents, etc., and various lysing agents Enzymes of components in the cell wall or cell membrane, such as Labiase lyase, lysostaphin, lysozyme from egg protein, human lysozyme, achromopeptidase, mutanolysin from Streptomyces coccidioides, chitinase, lysozyme Rhizoctonia-derived lyase, Arthrobacter luteus-derived lyase, Trichoderma harzianum-derived lyase, Streptococcus pyogenes-derived streptolysin O, Bacillus tetani-derived tetanolysin, etc. The main function of described nucleic acid extraction reagent is: (1) utilize detergent to destroy lipid bilayer, rupture cell; (2) dissolve protein; (3) promote protein denaturation; (4) inhibit protease and nuclease active. Commercially available direct extraction nucleic acid extraction reagents, such as the sample release agent (model S1014) provided by China Sunshine Biotechnology Co., Ltd.

In an exemplary working situation, the initial state of the chip device for nucleic acid detection in a sample is: the sampling valve 104 and the mixing chamber valve 106 are in a closed state; lyophilized direct extraction nucleic acid extraction reagents are preloaded in the first mixing chamber 105. When the sampling chamber valve 102 is closed and there is a sample solution in the sample loading chamber 103, the sampling valve 104 is opened, and the piston of the sample loading chamber 103 is moved downward, and the sample solution in the sample loading chamber 103 is pressed to the first mixing chamber 105, dissolve the preloaded direct extraction reagents; close the injection valve 104. Then, the mixing chamber valve 106 is opened, and the pistons of the first mixing chamber 105 and the second mixing chamber 107 are alternately moved downward, so that the direct extraction nucleic acid extraction reagents are fully mixed and reacted with the sample solution, and the cells in the sample are lysed, The nucleic acid is released, and finally the piston of the first mixing chamber 105 moves downward, so that the solution containing the released and isolated nucleic acid flows into the second mixing chamber 107, and the valve 106 of the mixing chamber is closed.

In the chip device for nucleic acid detection in samples provided by the present invention, the nucleic acid extraction module further includes a dilution chamber 109. The diluting chamber 109 is a piston-type container with a piston in the chamber and an opening communicating with the flow channel at the bottom of the chamber. There is also a dilution valve 108 between the second mixing chamber 107 and the diluting chamber 109, which is a piston valve with a piston in the valve chamber and two openings at the bottom of the valve chamber that communicate with the flow channel in the substrate, wherein the flow channels connected to the two openings communicate with the second mixing chamber 107 and the diluting chamber 109 respectively.

In the chip device for nucleic acid detection in this embodiment, after the nucleic acid is extracted from the sample, it usually needs to be diluted before being used for subsequent nucleic acid detection reactions, such as amplification reactions.

In an exemplary working situation, the initial state of the chip device for nucleic acid detection in a sample is: The dilution valve 108 is in a closed state; the piston of the dilution chamber 109 is located at a specified height, a dilution solution for diluting the extracted nucleic acid solution is preloaded in the chamber formed by the piston and the bottom. When there is a solution containing the isolated nucleic acid in the second mixing chamber 107, the dilution valve 108 is opened, the piston of the second mixing chamber 107 moves downward, and the piston of the dilution chamber 109 moves upward, so that the solution of separated nucleic acids in the second mixing chamber 107 flows to the dilution chamber 109. Optionally, the pistons of the second mixing chamber 107 and the diluting chamber 109 are moved downward alternately, so that the nucleic acid sample solution is fully mixed. Finally, the piston of the second mixing chamber 107 moves downward, so that the nucleic acid-containing solution flows into the diluting chamber 109, and the diluting valve 108 is closed.

In the chip device for detecting nucleic acid in a sample provided by the present invention, the nucleic acid extraction module further includes a filter unit and a sample-to-be-amplified chamber 110 downstream of it. In the present embodiment, the filter unit is a filter chamber 125, which is a piston-type container with a piston 126 in the chamber, and two openings that communicate with the dilution chamber 9 and the sample-to-be-amplified chamber 110 respectively at the bottom of the chamber; There is a filter element 127 at the bottom of filter chamber 125 for filtering unwanted substances (such as cells, cell fragments or large protein molecules, etc.) in the nucleic acid extraction solution. In the present invention, the filter element is prepared by using filter materials. In one aspect of the present invention, the piston in the filter chamber is fixed in the chamber, and the space formed by the piston and the bottom of the chamber is filled with the filter material. The pore size of the filter material can allow the nucleic acid in the solution (including genomic nucleic acid or its fragments, etc.) to pass through freely, and retain tissue fragments, cells and cell fragments, or large protein molecules, etc. In the present invention, the filter material used does not physically adsorb or substantially does not physically adsorb the nucleic acid in the solution, and does not react with the nucleic acid or cause inhibition of amplification reactions.

In an exemplary working situation, the initial state of the chip device for nucleic acid detection in a sample is: the piston 126 of the filter chamber 125 is fixed in the chamber and pressed on the top of the filter element 127; and the piston in sample-to-be-amplified chamber 110 is located at the bottom of the chamber. When the dilution valve 108 is closed and there is a solution containing the separated and diluted nucleic acid in the dilution chamber 109, the piston in the dilution chamber 109 moves downward, and the piston in the sample-to-be-amplified chamber 110 moves upward, thereby the separated and diluted nucleic acid solution in the cavity 109 flows through the filter material 127 between the two openings of the filter cavity 110 and then flows into the sample-to-be-amplified chamber 110.

In another embodiment of the present invention, a filter membrane may be provided in the flow channel between the second mixing chamber 107 and the sample-to-be-amplified chamber 110. In yet another embodiment of the present invention, a plurality of filter chambers or filter membranes or a combination thereof may be arranged between the second mixing chamber 107 and the sample-to-be-amplified chamber 110, and the multiple filter chambers or filter membranes may have different size of the sieve to realize the multiple filtration of the nucleic acid-containing solution obtained after the nucleic acid extraction of the sample by the direct extraction method, which can avoid the clogging of the filter material.

In another embodiment of the present invention, the chip device provided by the present invention for detecting nucleic acid in a sample does not need a filter chamber.

As shown in Fig 2 and Fig 3, the chip device for nucleic acid detection in samples provided by the present invention also has a nucleic acid amplification module. In the illustrated exemplary device, the nucleic acid amplification module has a multiple amplification unit, and the amplification unit consists a plurality of amplification reaction chambers 122 arranged in the substrate of the chip device and a multiple amplification loading valve 121 above said plurality of amplification reaction chambers 122. As shown in Figure 2, the multiple amplification loading valve 121 is a piston valve, with a piston 123 in the valve chamber, and an opening 124 in the center of the bottom of the valve chamber communicating with the upstream sample-to-be-amplified chamber and a plurality of openings 125 provided in the circumference of the bottom of the valve chamber connected to the flow channels each connecting with one of the plurality of amplification reaction chambers 122. The plurality of amplification reaction chambers 122 are arranged in the substrate of the chip below the valve chamber of the multiple amplification sampling valve 121, which can accommodate the reactants or reaction systems of the amplification reaction, and the isolated nucleic acid solution from the nucleic acid extraction module. The isolated nucleic acid solution can be subjected to an amplification reaction under suitable conditions. In other aspects of the present invention, the nucleic acid amplification module in the chip device may have multiple nucleic acid amplification units, such as 2 or 4 or 6 or 8 units.

In the present invention, multiple amplification (or multi-probe recognition) can be used to detect multiple target sequences on the nucleic acid of the same sample under the same reaction conditions. For example, through multiplex PCR (multiplex PCR), that is, a PCR reaction in which different primer pairs are used in the same PCR reaction system to simultaneously amplify multiple nucleic acid fragments. By arranging multiple amplifications and detections of the nucleic acid of the same sample respectively in multiple amplification reaction chambers of the device provided by the present invention, rapid and efficient detection of the sample can be conveniently realized.

In an exemplary working situation, the initial state of the chip device for nucleic acid detection in a sample is: different primer pairs, probes, one or more of reactants such as enzymes are preloaded in the plurality of amplification reaction chambers 122 (in another exemplary working situation, the enzyme is preloaded in the sample-to-be-amplified chamber 110, and is dissolved in the solution after adding the diluted nucleic acid sample solution), and kept in a vacuum state; the piston of the multiple amplification sampling valve 121 is located at the bottom of the chamber, sealing the opening 124 communicating with the flow channel connected to the upstream sample-to-be-amplified chamber 110 and the opening 125 communicating with each of the multiple amplification reaction chambers 122. When the dilution valve 108 is closed and there is a diluted nucleic acid sample solution in the sample-to-be-amplified chamber 110, the piston of the multiple amplification sampling valve 121 is moved upwards, thereby making the nucleic acid sample solution in the sample-to-be-amplified chamber 110 enters the chamber of the multiple amplification sampling valve 121 and then is evenly dispensed into the multiple amplification reaction chambers 122.

Further, nucleic acid amplification reactions can be performed in the plurality of amplification reaction chambers 122. Various nucleic acid amplification methods using primers known in the art can be used in the present invention, including variable temperature or isothermal amplification methods, such as polymerase chain reaction (PCR), strand displacement amplification (SDA), nucleic acid sequence-based Amplification (NASBA), cascade rolling circle amplification (CRCA), loop-mediated DNA isothermal amplification (LAMP), isothermal and chimeric primer-initiated nucleic acid amplification (ICAN), target-based unwinding Enzyme-dependent amplification (HDA), transcription-mediated amplification (TMA), etc.

In one of the embodiments of the present invention, a temperature-changing amplification method such as PCR is used, and the nucleic acid amplification module of the device also includes a temperature control unit for the plurality of amplification reaction chambers 122, for example, a temperature regulator, the amplification reaction chamber 122 is heated and cooled according to a predetermined program and lasts for a predetermined time.

In another embodiment of the present invention, using an isothermal amplification method such as LAMP, the nucleic acid amplification module of the device also includes a temperature control unit for the plurality of amplification reaction chambers 122, for example, a temperature regulator, so that the amplification reaction chamber 122 maintains a constant temperature.

The chip device for detecting nucleic acid in a sample provided by the present invention may also include a signal detection module (not shown in the figure) for detecting the detection results of each amplification reaction chamber 122. The detection module of the present invention is a module suitable for detecting identifiable labels carried by nucleic acids in any manner, including but not limited to fluorescence or other forms (such as chemiluminescence, bioluminescence, radioluminescence, electroluminescence, electrochemiluminescence, mechanical luminescence, crystalline luminescence, thermoluminescence, sonoluminescence, phosphorescence and photoluminescence, etc.) luminescence, enzymatic reactions, radioactivity, etc.

In one aspect of the present invention, the amplification reaction chambers of the amplification reaction unit are insulated by materials that completely absorb or substantially completely absorb the signals generated by the amplification reaction, so as to avoid the interference caused by adjacent amplification reaction chambers. For example, the wall of the amplification reaction unit can be made of a material that completely absorbs or substantially completely absorbs the signal generated by the amplification reaction. At the same time, the bottom of the amplification reaction chamber is prepared or sealed with a material that does not absorb at all or substantially does not absorb the signal to be detected. Thus, the signals generated by nucleic acid amplification in each amplification reaction chamber can be effectively detected through the bottom of the amplification reaction chamber.

In one aspect of the present invention, the detection of the amplified nucleic acid is by detecting the fluorescent signal carried by the nucleic acid. The probes, primers and oligonucleotides contained in the reaction system of the amplification reaction chamber 122 can be detectably labeled radioactively, fluorescently or non-radioactively by methods well known to those skilled in the art. The bottom of the amplification reaction chamber 122 is prepared or sealed with a material that does not absorb at all or substantially does not absorb the signal to be detected. Thus, the signal detection module disposed in the chip or an external detection system can detect the fluorescent signals generated in each amplification reaction chamber 122 through the bottom of the amplification reaction chamber 122. Commonly used fluorescent dyes and their signal-related wavelengths are shown in Table 1 below.

**Table 1 Fluorescent Dyes**

| Dye | Excitation wavelength | emission wavelength |
|---|---|---|
| FAM/SYBGREEN | 492nm | 516nm |
| HEX/JOE/VIC | 535nm | 555nm |
| ROX/TEXRED | 585nm | 610nm |
| CY5 | 635nm | 665nm |

In one aspect of the present invention, the bottom of the amplification reaction chamber 122 can be made of a material that does not absorb the fluorescent signal generated by the amplification reaction. In addition, various known optical systems (including optical filters, cameras, etc.) can be used to collect and analyze the fluorescence signals generated at the bottom of each amplification reaction chamber 122 to obtain detection results.

In the chip device for detecting nucleic acid in a sample provided by the present invention, the piston-style containers are used to accommodate solid or liquid preparations or to conduct reactions. Liquid formulations can be transferred between interconnected containers through fluid channels. The solid formulation can be formed into a solution after addition of a suitable solvent, and then transferred between interconnected containers through fluid channels.

In the chip device for nucleic acid detection in samples provided by the present invention, two or more piston-style containers can be used to control the flow of fluid (stationary or moving), as well as its flow direction and/or flow rate. In addition, the liquid can be mixed effectively through two or more piston-style containers connected. For example, as mentioned above, the pistons of the first mixing chamber 105 and the second mixing chamber 107 can be moved down alternately, so that the direct extraction nucleic acid extraction reagent and the sample solution are fully mixed and reacted.

In the chip device for nucleic acid detection in samples provided by the present invention, the movement of the piston in the cavity can be controlled by mechanical transmission. For example, the piston is fixedly connected with the connecting rod, and the position of the piston in the chamber is controlled by pushing and pulling the connecting rod. When the piston moves to the bottom of the chamber, it can continue to apply pressure, thereby sealing the opening where the fluid channel communicates with the chamber, and completely blocking the fluidic communication between the fluid channels. In other embodiments of the present invention, the movement of the piston can also be controlled by other means, such as pneumatic transmission or hydraulic transmission.

In one embodiment, in the chip device for nucleic acid detection in samples provided by the present invention, the movement of the piston in the piston valve in the chamber can be controlled by a piston movement control mechanism. As shown in FIG. 1, in the piston valve 8, the piston movement control mechanism includes a piston movement control member 82 arranged above the piston 81 in the chamber, the piston movement control member 82 has an external thread, and forms a thread pair with the inner thread on the chamber wall of the piston, and when the piston movement control member 82 is rotated along the thread, it moves upward or downward in the chamber of the piston, thereby controlling the upward or downward movement of the piston.

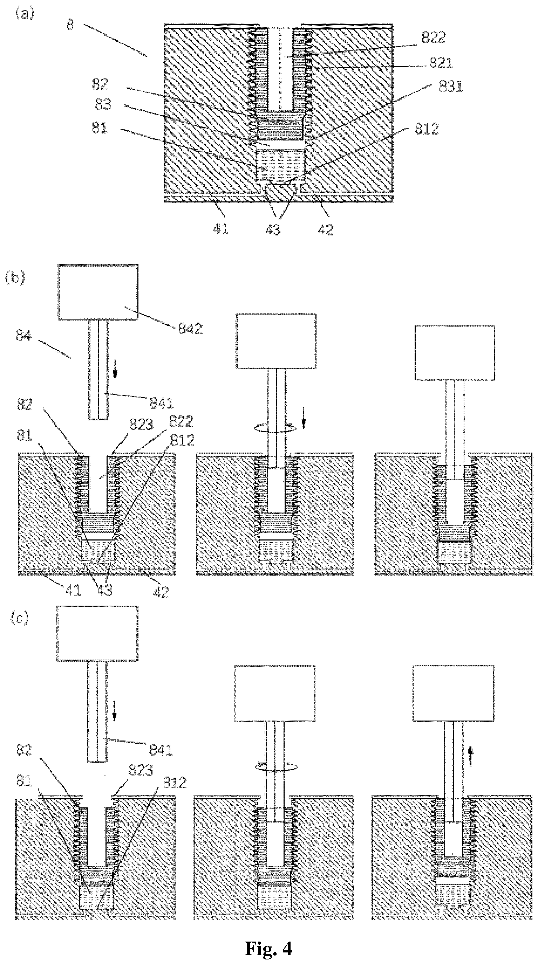

FIG. 4 is a schematic diagram of the structure and working mode of the aforementioned exemplary piston valve and piston motion control mechanism in the chip device provided by the present invention. FIG. 4 (a) is a schematic cross-sectional view of the structure of the exemplary piston valve, and FIG. 4 (b) and (c) are schematic views of the working mode of the exemplary piston valve. In the exemplary piston valve 8 shown in FIG. 4 (a), the piston 81 is located in a cavity 83 formed by the space limited by the cavity wall, and can move up and down in the cavity. The piston is movable into full contact with the bottom of the cavity and covers the opening 43 through which all fluid channels (e.g., fluid channels 41 and 42 as shown) communicate with the cavity, thereby blocking fluidic communication. The piston movement control member 82 is arranged above the piston 81 and has an external thread 821 forming a thread pair with the internal thread 831 on the cavity wall of the piston valve. As the piston motion control member 82 is moving around along the thread, it moves upward or downward within the cavity of the piston. FIG 4(b) and (c) are schematic diagrams of the working mode of the exemplary piston valve, wherein FIG 4(b) shows an exemplary working mode of controlling the downward movement of the piston, and FIG 4(c) shows an exemplary working mode of controlling the upward movement of the piston. In the exemplary piston valve shown in FIG 4 (b) and (c), the movement of the piston motion control member 82 is controlled by the control mechanism 84 of the piston motion control member. As shown in FIG. 4(b), the control mechanism includes a control rod 841 and a motor 842. The control rod can be rotated and/or moved up and down. In one of the implementations, the control rod movement motor can be an electric batch, which can set parameters such as the rotation direction, rotation speed, rotation angle, and stop torque of the control rod. The piston movement control member 82 has a cavity 822 suitable for inserting the control rod, and the cross section of the cavity is in a shape adapted to the control rod (such as the hexalobe screw in the embodiment shown in FIG. 4(b)). FIG. 4(b) is a schematic diagram of controlling the downward movement of the piston movement control member. As shown in FIG 4 (b), after the control rod 841 is inserted into the cavity of the piston motion control part, the clockwise rotation of the control rod can drive the piston motion control part 82 to rotate along the screw thread, and the piston motion control part 82 moves downward in the cavity. In one aspect of the invention, the control rod is insertable in a slowly rotating manner into the cavity of the piston motion control member. Inserting while rotating allows the ribs on the rod to engage with the interior of the cavity, effectively solving the alignment problem. In the exemplary piston mechanism shown in FIG. 4, there is no fixed connection between the piston motion control member 82 and the piston 81. The bottom of the piston has a piston supporting body 812, which is a protrusion at the bottom of the piston, made of elastic material, and will be deformed and compressed when subjected to pressure. When the piston is under pressure (for example, when the piston motion control member rotates along the thread and moves toward the piston in the cavity, contacts the piston and pushes it to the bottom of the cavity), the piston support body 812 is compressed to the entire bottom of the piston and fully contacts the bottom of the cavity and covers the opening 43 that all fluid passages ( fluid passages 41 and 42 as shown in the figure) communicating with the cavity, thus blocking the fluidic communication of each fluid passage (as shown in FIG 4 (b)). When the received pressure of the rod disappears (for example, when the piston motion control member moves away from the piston in the cavity), the piston support body 812 returns to its natural form state (that is, the piston support body under no additional pressure on the piston), in this state, the piston support body contacts the bottom of the piston but does not cover any opening 43 where any fluid channel communicates with the cavity, and each fluid channel communicates with each other through the cavity. The movement distance of the control rod is used to adjust the contact and compression of the piston motion control part 82 and the piston 81. At the same time, a limit 823 to the piston motion control part can be set at the top area of the chamber wall, so that when the piston motion control part moves to the limit, the torque received by the control rod in the piston movement control part exceeds the stop torque value set by the electric batch, the electric batch stops the rotation of the motor, and then the control rod can be pulled out.

FIG. 4 (c) is a schematic diagram of controlling the upward movement of the piston movement control member. As shown in FIG. 4 (c), after the control rod 841 is inserted into the cavity, the counterclockwise rotation of the control rod can drive the piston movement control member 82 to rotate along the thread, and the piston movement control member moves upward in the cavity. When the pressure on the piston 81 disappears (for example, when the piston motion control part does not contact the piston), the piston support body 812 returns to its natural state (that is, the natural deformation of the piston support body under no additional pressure on the piston), in this state, the part of the piston support body in contact with the bottom of the piston does not cover any opening where the fluid channel communicates with the cavity, and each fluid channel communicates with each other through the cavity. When the piston movement control part moves to the top, because of the existence of the limit position 823, the torque increases. After exceeding the stop torque value set by the electric batch, the electric batch will stop the rotation of the control rod, and then the control rod can be pulled out.

In one embodiment, in the chip device for nucleic acid detection in samples provided by the present invention, the initial working state of the piston in the aforementioned piston container is located at a specified height in the chamber. For example, the piston in the sample loading chamber 103 in FIG. 2 is at a specified height in the initial working state (at this time, the volume between the piston and the bottom of the sample loading chamber is a specified volume, such as the specified sample volume for the sample extraction reaction). The positioning of the piston in the chamber can be achieved by increasing the friction between the piston and the piston wall by etching internal threads on the piston wall or the like. As shown in FIG. 1, the chamber wall of the piston container 3 has an internal thread 31, and the lower end of the internal thread is the upper end of the predetermined piston position.

### Example 2

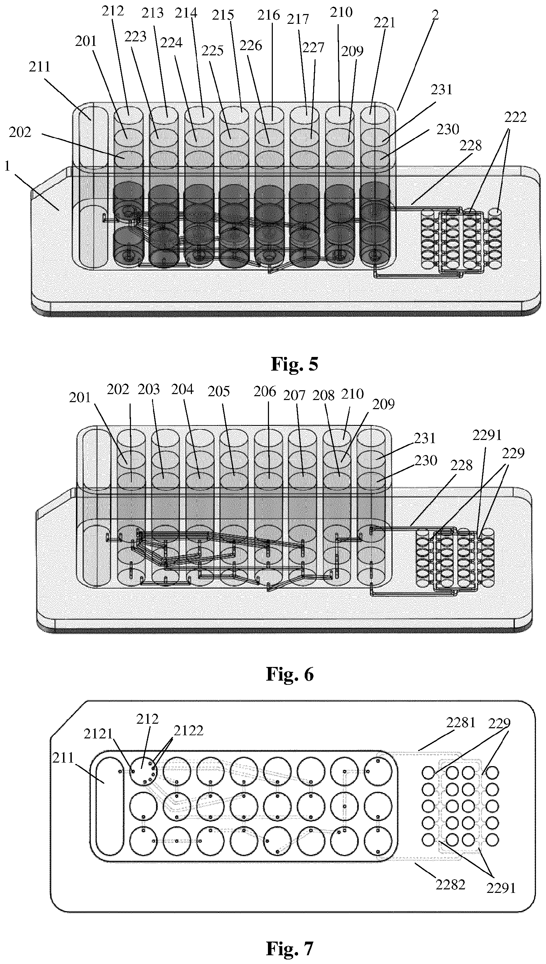

FIG. 5 is a perspective view of yet another exemplary chip device used in the detection of nucleic acid in a sample provided by the present invention. The figure does not show the internal thread on the chamber wall of some piston-style containers or piston valves in the chip device provided by the present invention and the piston movement control parts in the chamber of the piston valve. FIG. 6 is another perspective view of the exemplary chip device for nucleic acid detection in a sample shown in FIG. 5. The figure does not show the piston in the piston container or the piston valve in the chip device provided by the present invention. FIG. 7 is a schematic diagram of the top-view structure of the exemplary chip device for detecting nucleic acid in a sample shown in FIG. 5 and the communication relationship of its flow channels.

As shown in FIG. 5, the chip device for nucleic acid detection in samples provided by the present invention includes a substrate 1 and a container area 2 perpendicular to the substrate, and a plurality of cylindrical containers 3 are arranged in the container area. The cylindrical chamber is formed with wall of the container area perpendicular to the bottom plate. The cylindrical chambers communicate with each other through fluid channels provided in the bottom plate. The fluid channel is located below the chamber, and communicates with the opening at the bottom of the cavity through an upward flow channel.

As shown in FIG 5 and FIG 6, the chip device used for nucleic acid detection in samples provided by the present invention has a sample receiving module, including a sample receiving chamber 201 and a sample loading chamber 203, and a sampling cavity valve 202. The sample to be tested can be added into the sample receiving chamber. When the concentration of the sample to be tested is greater than the specified concentration, water or other suitable solutions such as PBS buffer can be added to the sample loading chamber. The sampling chamber valve 202 is a piston valve with a piston inside the valve chamber and two openings at the bottom of the valve chamber that communicate with the flow channel in the substrate. The flow channels communicate with the sample receiving chamber 201 and the sample loading chamber 203 respectively. The sampling cavity 203 is a piston-type container with a piston in the cavity and an opening at the bottom of the cavity that communicates with the flow channel in the substrate. In an exemplary working situation, the initial state of the device for nucleic acid detection in a sample is: the sampling cavity valve 202 is in a closed state (that is, the piston of the sampling cavity valve 202 is located at the bottom of the cavity and covers the opening, blocking the cavity communication with the fluid channel), the piston in the sample loading chamber 203 is at a specified height (the volume between the piston and the bottom of the sample loading chamber is a specified volume at this time, for example, the specified sample volume for the sample cracking reaction). When keeping the sample loading chamber valve 202 in the closed state, add the sample in the sample receiving chamber 201; then the sampling chamber valve 202 is opened (the piston of the sampling chamber valve 202 is raised), because the initial state of the sample loading chamber 203 is a vacuum state, the solution in the sample receiving chamber 201 of the specified volume is sucked into the sample loading chamber 203 through the sampling chamber valve 202; the sampling chamber valve 202 is then closed (the piston of the sampling chamber valve 202 is pushed to the bottom of the chamber).

As shown in FIG. 5 and FIG. 6, the chip device used for nucleic acid detection in samples provided by the present invention has a sample lysis and nucleic acid extraction module, which includes a first mixing chamber 205 and a second mixing chamber 207, and there is a mixing chamber valve 206 between said first mixing chamber 205 and said second mixing chamber 207, which is a piston valve. There is also a sampling valve 204 between the sample loading chamber 203 and the first mixing chamber 205. Wherein, the first mixing chamber 205 and the second mixing chamber 207 are both piston-type containers, with a piston inside the chamber. The sampling valve 204 and the mixing chamber valve 206 are both piston valves, with a piston in the valve chamber, and two openings at the bottom of the valve chamber that communicate with the flow channel in the substrate, and the two openings are respectively connected with flow channels of the upstream and downstream the containers: the two flow channels controlled by the sampling valve 204 communicate with the first mixing chamber 205 and the sample loading chamber 203 respectively, and the two flow channels controlled by the mixing chamber valve 206 communicate with the first mixing chamber 205 and the second mixing chamber 207 respectively.

The chip device for nucleic acid detection in this embodiment is suitable for nucleic acid extraction after lysing the sample by lysing method, that is, after the sample contacts and reacts with the sample lysing reagent, it needs to be further processed by nucleic acid extraction methods such as magnetic bead method to obtain usable Nucleic acid in subsequent amplification reactions. In the magnetic bead method, the nucleic acid is bound by contacting the nucleic acid-binding magnetic material and/or binding solution with the sample that has been lysed, thereby allowing the nucleic acid to bind to the magnetic material. The complex formed after the nucleic acid is combined with the magnetic material can controllably move, stir or precipitate in the container under the action of a magnetic field, so as to achieve the purposes of nucleic acid binding, nucleic acid cleaning and nucleic acid elution. The nucleic acid extraction module in the chip device provided by the present invention is composed of a binding unit, a cleaning unit and an elution unit, and nucleic acid is extracted through nucleic acid binding, cleaning and elution steps, wherein nucleic acid binding, nucleic acid cleaning and nucleic acid elution reagents need to be added (including nucleic acid extraction magnetic beads, washing solution, eluent, etc.).

As shown in FIG. 5 and FIG. 6, the chip device for detecting nucleic acid in a sample provided by the present invention has a sample lysis and nucleic acid extraction module, which includes a sample lysis module and a nucleic acid extraction module. Wherein, the sample lysing module includes a lysing reagent storage chamber 215 for accommodating a lysing reagent (solid), and a lysing reagent valve 225 located between the first mixing chamber 205 and the lysing reagent storage chamber 215. The lysing reagent valve is a piston valve, comprising a valve chamber and a valve piston positioned in the valve chamber. The piston can move to the bottom of the chamber and cover the openings, blocking the fluidic communication between the lysis reagent storage chamber and the chamber where sample lysis is performed (e.g., the first mixing chamber 205). And, wherein said nucleic acid extraction module comprises three nucleic acid cleaning reagent storage chambers 213, 214 and 216 that accommodate nucleic acid cleaning reagents (solid), and acid cleaning reagent valves 223, 224, 226 between the first mixing chamber 205 and each of said nucleic acid cleaning reagent storage chambers 213, 214 and 216. And, the nucleic acid extraction module also includes a nucleic acid elution reagent storage chamber 217 for holding a nucleic acid elution reagent (solid), and a nucleic acid cleaning reagent valve 227 between the first mixing chamber 205 and the nucleic acid elution reagent storage cavity 217.

As shown in FIG. 5 and FIG. 7, the sample lysis and nucleic acid extraction module of the chip device for nucleic acid detection in samples provided by the present invention also includes a water adding chamber 211 and a water adding valve 212. Wherein, the water adding valve 212 is a piston valve, has a piston in the valve chamber, and has an opening 2121 communicated with the water adding chamber 211 at the bottom of the valve chamber, and five openings 2122 communicating with the lysing reagent storage chamber 215 and the nucleic acid cleaning reagent storage chamber 213, 214, 216 the nucleic acid eluting reagent storage chamber 217.

In an exemplary working situation, the initial state of the chip device for nucleic acid detection in a sample is: the water adding valve 212, the lysing reagent valve 225, the nucleic acid cleaning reagent valves 223, 224, 226 and the nucleic acid cleaning reagent valve 227 are closed. When starting to work, add distilled water to the water adding chamber 211, open the water adding valve 212, and the distilled water in the water adding chamber 211 will be sucked into the lysing reagent storage chamber 215, nucleic acid cleaning reagent storage chambers 213, 214, 216 and nucleic acid eluting reagent storage chambers 217, and resuspend the reagents therein. Then close the water adding valve 212. At the same time, the initial state of the chip device for nucleic acid detection in the sample is: the sampling valve 204 and the mixing chamber valve 206 are closed; the second mixing chamber 207 is preloaded with magnetic beads that can bind nucleic acid. When the sampling chamber valve 202 is closed and there is a sample solution in the sample loading chamber 203, open the sampling valve 204 to move the piston of the sample loading chamber 203 downward, and press the sample solution in the sample loading chamber 203 to the first mixing chamber 205; close sampling valve 204. Then, open the lysis reagent valve 225, press down the piston of the lysis reagent storage chamber 215, press the lysis reagent solution into the first mixing chamber 205, and then close the lysis reagent valve 225. Open the mixing chamber valve 206, and alternately move the pistons of the first mixing chamber 205 and the second mixing chamber 207 downward, so that the sample solution, the lysing reagent solution and the magnetic beads bound to nucleic acid are fully mixed and reacted, and the cells in the sample are lysed, the nucleic acid is released and bound to the magnetic beads. Place a magnetic device such as a magnet close to the first mixing chamber 205 and/or the second mixing chamber 207, so that the magnetic beads are attracted on the chamber wall, and press down the piston of the second mixing chamber 207 to the bottom, so that all the liquid is discharged to the first mixing chamber 205, then close the mixing chamber valve 206, open the sampling valve 204 and the extraction reagent valve 225, press down the piston of the first mixing chamber 205, so that the waste liquid enters the sample loading chamber 203 and the extraction reagent storage chamber 215, and then the sampling valve 204 and the lysing reagent valve 225 are closed, thereby completing the lysing of the sample and the binding reaction in nucleic acid extraction.

Open the nucleic acid cleaning reagent valve 223, press down the piston of the nucleic acid cleaning reagent storage chamber 213, press the nucleic acid cleaning reagent for the first nucleic acid cleaning into the first mixing chamber 205, and then close the nucleic acid cleaning reagent valve 223. Removing the magnetic device, such as a magnet, allows the magnetic beads to dislodge from the chamber walls and disperse in the nucleic acid washing reagent solution. Open the mixing chamber valve 206, and alternately move the pistons of the first mixing chamber 205 and the second mixing chamber 207 downward, so that the magnetic beads are fully mixed and washed in the nucleic acid cleaning reagent. Place a magnetic device such as a magnet close to the first mixing chamber 205 and/or the second mixing chamber 207, so that the magnetic beads are attracted on the chamber wall, and press down the piston of the second mixing chamber 207 to the bottom, so that all the liquid is discharged to the first mixing chamber 205, then close the mixing chamber valve 206, open the nucleic acid cleaning reagent valve 223, press down the piston of the first mixing chamber 205, so that the waste liquid enters the nucleic acid cleaning reagent storage chamber 213, then close the nucleic acid cleaning reagent valve 223, Thus, the first washing of the magnetic beads adsorbed with nucleic acid is completed.

The same operation is performed on the nucleic acid cleaning reagent storage chambers 214 and 216 in sequence to complete the second and third cleaning of the magnetic beads adsorbed nucleic acid.

Open the nucleic acid cleaning reagent valve 227, press down the piston of the nucleic acid eluting reagent storage chamber 217, press the nucleic acid eluting reagent into the first mixing chamber 205, and then close the nucleic acid cleaning reagent valve 227. Removing the magnetic device, such as a magnet, causes the magnetic beads to dislodge from the chamber walls and disperse in the nucleic acid elution reagent solution. Open the mixing chamber valve 206, and alternately move the pistons of the first mixing chamber 205 and the second mixing chamber 207 downward, so that the magnetic beads are fully mixed and reacted in the nucleic acid elution reagent. A magnetic device such as a magnet is placed close to the first mixing chamber 205 and/or the second mixing chamber 207, so that the magnetic beads are attracted on the wall of the chamber, and finally the piston of the first mixing chamber 205 moves downward, so that the solution containing the nucleic acid isolated flows into the second mixing chamber 207, and the mixing chamber valve 206 is closed.

In another aspect of the present invention, all or part of the components in the sample lysing reagent (such as enzymes) can be preloaded in the first mixing chamber 205.

In another aspect of the present invention, the lysing reagent, nucleic acid cleaning reagent and nucleic acid eluting reagent of the lysing reagent (solid) in the sample lysing and nucleic acid extraction module in the chip device for nucleic acid detection in the sample of the present invention can also be liquid form preloaded in the lysing reagent storage chamber, the nucleic acid washing reagent storage chamber or the nucleic acid eluting reagent storage chamber.

In the chip device for detecting nucleic acid in a sample provided in this embodiment, the nucleic acid extraction module further includes a sample-to-be-amplified mixing chamber 209 and a sample-to-be-amplified chamber 210. The sample-to-be-amplified mixing chamber 209 and the sample-to-be-amplified chamber 210 are piston-type containers with a piston inside the chamber and an opening at the bottom of the chamber communicating with the flow channel. Between the second mixing chamber 207 and the sample-to-be-amplified mixing chamber 209, there is also a sample-to-be-amplified valve 208, which is a piston valve with a piston in the valve chamber and two openings communicate with a flow channel in the substrate at the bottom of the valve chamber communicate with the second mixing chamber 207 and the sample-to-be-amplified mixing chamber 209 respectively.

In an exemplary working situation, the initial state of the chip device for nucleic acid detection in a sample is: the sample-to-be-amplified valve 208 is in a closed state; reagents (such as common amplification reagents such as enzymes or buffer reagents, etc., but not including reagents for specific amplification, such as specific primers or probes) for the following detection reactions (such as amplification) are preloaded in the sample-to-be-amplified mixing chamber 209 and/or the sample-to-be-amplified chamber 210. Under the situation that there is the solution that contains the separated nucleic acid in the second mixing chamber 207, open the sample-to-be-amplified valve, the piston of the second mixing chamber 207 moves downward, and the piston of the sample-to-be-amplified mixing chamber 209 moves upward, so that the solution of the separated nucleic acid in the second mixing chamber 207 flows to the sample-to-be-amplified mixing chamber 209. Optionally, the piston of the sample-to-be-amplified chamber 210 is moved upwards at the same time, so that the solution of the separated nucleic acid flows to the sample-to-be-amplified chamber 210; close the sample-to-be-amplified valve 208; alternately move the pistons of sample-to-be-amplified mixing chamber 209 and the sample-to-be-amplified chamber 210 downward, so that the nucleic acid sample solution is fully mixed. Finally, the piston of sample-to-be-amplified mixing chamber 209 moves downward, so that the solution containing the nucleic acid flows into the sample-to-be-amplified chamber 210.

As shown in FIG. 5 and FIG. 6, the chip device for nucleic acid detection in a sample provided by the present invention also has a nucleic acid amplification module. In the illustrated exemplary chip device, the nucleic acid amplification module includes a plurality of amplification reaction chambers 222 arranged in an array set in the substrate of the chip device, wherein each amplification reaction chamber 222 communicates with branch channels 2291 between every two rows of said amplification reaction chambers through a sub-branch channel 229, and multiple sub-branch channels 2291 merge at both ends of the amplification reaction chamber array to form a main channel 2281. The main channel 2281 at one end of the amplification reaction chamber array communicates with the upstream multiple amplification sampling valve 221. The multiple amplification sampling valve 221 is a piston valve with a piston in the valve chamber and two openings at the bottom of the valve chamber, wherein the two openings communicate with the flow channels in the substrate connecting the sample-to-be-amplified chamber 210 and the main flow channel 2281.

In the present invention, an inert liquid such as mineral oil can be used to seal the outlet of the chamber for nucleic acid amplification reaction. In one embodiment of the present invention, the nucleic acid amplification module of the chip device for nucleic acid detection in samples provided by the present invention further includes an inert liquid module for storing the inert liquid and transferring the inert liquid. The inert liquid module has an oil filling chamber for storing and providing an inert liquid (such as mineral oil or paraffin oil) that seals the interface between the amplification reaction chamber and the branch flow channel; optionally, it also includes an oil filling valve between the oil filling chamber and the multiple amplification zone units, which is a piston valve with a piston in the valve chamber, and at the bottom of the valve cavity, there is an opening communicating with the flow channel of the oil filling chamber and an opening communicating with the amplification zone unit. As shown in FIG. 5, FIG. 6 and FIG. 7, the inert liquid module includes an oil filling chamber 231. There is also an oil filling valve 230 between the main channel 2282 at the other end of the amplification reaction chamber array and the oil filling chamber 231. The oil filling valve 230 is a piston valve with a piston in the valve chamber and two openings at the bottom communicating with the flow channels in the substrate connected with the oil filling chamber 231 and the main flow channel 2282 respectively.

In an exemplary working situation, the initial state of the chip device for nucleic acid detection in a sample is: different primer pairs, probes, one or more of reactants such as enzymes are preloaded in the plurality of amplification reaction chambers 222 (in another exemplary working situation, the enzyme is preloaded in the sample-to-be-amplified chamber 210, and is dissolved in the solution after adding the diluted nucleic acid sample solution), and kept in a vacuum state; the piston of the multiple amplification sampling valve 221 is located at the bottom of the chamber, sealing the opening communicating with the flow channel connected to the upstream sample-to-be-amplified chamber 210 and the opening communicating with the main channel 2281. When the dilution valve 208 is closed and there is a diluted nucleic acid sample solution in the sample-to-be-amplified chamber 210, the piston of the multiple amplification sampling valve 221 is moved upwards, thereby making the nucleic acid sample solution in the sample-to-be-amplified chamber 210 enters the chamber of the multiple amplification sampling valve 221, and then enter sub-branch channel 229 through main channel 2281 and sub-branch channels 2291, and then is evenly dispensed into the multiple amplification reaction chambers 222.

In addition, in an exemplary working situation, the initial state of the chip device for nucleic acid detection in a sample is: the oil adding valve 230 is in a closed state; the oil adding chamber 231 is pre-filled with mineral oil. After the reaction systems have been divided into the multiple amplification reaction chambers 222, open the oil adding valve 230, press down the piston of the oil adding chamber 231, and the mineral oil present in the oil adding chamber 231 will pass through the oil adding valve 230 and enter the main channel 2282. In the process, the mineral oil passes through the branch flow channel 2291, closes each sub-branch flow channel 229, and then passes through the main flow channel 2281 and flows through the multiple amplification sampling valve 221 into the sample-to-be-amplified chamber 210 and sample-to-be-amplified mixing chamber 209. Close the oil filling valve 230 and the multiple amplification sampling valve 221. The mineral oil pre-stored in the oil adding chamber 231 is excessive, which can ensure that the connections between the various amplification reaction chambers 222 are separated by the oil.

Nucleic acid amplification reactions can be performed in the plurality of amplification reaction chambers 222. As described in Example 1, various nucleic acid amplification methods using primers known in the art can be used in the present invention. In one embodiment of the present invention, a temperature-changing amplification method such as PCR is used. In another embodiment of the invention, an isothermal amplification method such as LAMP is used. The detection module used to detect each amplification reaction chamber 222 is a module suitable for detecting identifiable labels carried by nucleic acids in any manner, including but not limited to fluorescence or other forms of luminescence, enzymatic reactions, radioactivity, and the like.

As described in Example 1, In the chip device for detecting nucleic acid in a sample provided by the present invention, the piston-style containers are used to accommodate solid or liquid preparations or to conduct reactions. Liquid formulations can be transferred between interconnected containers through fluid channels. The solid formulation can be formed into a solution after addition of a suitable solvent, and then transferred between interconnected containers through fluid channels. In the chip device for nucleic acid detection in samples provided by the present invention, two or more piston-style containers can be used to control the flow of fluid (stationary or moving), as well as its flow direction and/or flow rate.

As described in Example 1, the piston valve in the chip device for nucleic acid detection in samples provided by the present invention can use the piston and the piston motion control mechanism as described in FIG. 4.

As described in Example 1, in the chip device for nucleic acid detection in samples provided by the present invention, the initial working state of the piston in the aforementioned piston container is located at a certain specified height in the chamber. The positioning of the piston in the chamber can be achieved by increasing the friction between the piston and the piston wall by etching internal threads on the piston wall or the like.

### Example 3 Instrument used for the nucleic acid detection in the sample

In one of the embodiments, the present invention provides an instrument for nucleic acid detection in a sample, which is a POCT instrument, which includes the chip device defined and described in Example 1 or 2.

The instrument has a chip device receiving and motion control system for receiving and transferring the above chip device to a designated position within the instrument for various processes on the chip.

The instrument has a magnet and a system to control the movement of the magnet, and is used to purify nucleic acid in a sample using the magnetic bead method. In the magnetic bead method, the nucleic acid is bound by contacting the nucleic acid-binding magnetic material and/or binding solution with the sample that has been lysed, thereby allowing the nucleic acid to bind to the magnetic material. The complex formed after the nucleic acid is combined with the magnetic material can be controlled to move, stir or precipitate in the container under the action of a magnetic field, so as to achieve the purposes of nucleic acid binding, nucleic acid cleaning and nucleic acid elution.

The instrument has a control mechanism for regulating the movement of a piston motion control in the piston valve. In one aspect of the present invention, the control mechanism includes a control rod and a control rod movement mechanism, the control rod can rotate or move up and down, wherein the control rod movement mechanism includes a component for controlling the control rod up and down movement and rotation, such as motors.

The instrument has a signal detection module for detecting nucleic acid amplification products, such as a fluorescence detection system.

The instrument has a temperature control system for the nucleic acid amplification area of the chip.

The instrument has a nucleic acid amplification result analysis and/or output system.

The above descriptions are only preferred embodiments of the present invention, and are not intended to limit the present invention. Any modifications, equivalent replacements, improvements, etc. made within the spirit and principles of the present invention shall be included in the scope of the present invention, within the scope of protection.

## Claims

1. A chip device for detecting nucleic acids in a sample, wherein said chip device has a substrate and piston-style containers, said piston-style containers being in communication with each other through a microfluidic channel, wherein each piston-style container comprises a chamber and a piston disposed within the chamber, the chamber having an opening in communication with said microfluidic channel at a bottom thereof, and
said chip device further has a piston valve disposed between the containers for controlling fluidic communication between interconnected containers, said piston valve comprising a valve chamber and a valve piston disposed within the valve chamber, the valve chamber having two or more openings in communication with a flow channel at a bottom thereof, the valve piston being configured to move to the bottom of the chamber for covering said openings, blocking communication of the chamber with the microfluidic channel, thereby blocking fluidic communication between containers connected through said microfluidic channel,
wherein said chip device comprises:
a sample receiving module;
a sample lysis and nucleic acid extraction module, used to perform a lysis reaction and/or a nucleic acid extraction reaction on the sample to obtain purified nucleic acids from the sample, and comprising a sample lysis module and/or a nucleic acid extraction module, and optionally, said nucleic acid extraction module comprising a nucleic acid binding unit, a nucleic acid washing unit, and a nucleic acid elution unit; and
a nucleic acid amplification module, for distributing nucleic acid samples to a plurality of amplification zone containers and for amplifying and/or detecting nucleic acid molecules, the nucleic acid amplification module comprising a multiplexed amplification zone module and, optionally, a preamplification zone module.

2. The chip device according to claim 1, **characterized in that** said sample-receiving module comprises a sample loading chamber, which is a piston-style container comprising a chamber and a piston disposed within the chamber; and
preferably, said sample-receiving module comprises a sample receiving chamber and a sample loading chamber, and further comprises a sample loading chamber valve between the sample receiving chamber and the sample loading chamber, which is a piston valve.

3. The chip device according to claim 1, **characterized in that** said sample lysis and nucleic acid extraction module comprises a first mixing chamber and a second mixing chamber, said first mixing chamber and said second mixing chamber each being a piston-style container comprising a chamber and a piston disposed within the chamber;
preferably, a mixing chamber valve is arranged between said first mixing chamber and said second mixing chamber, which is a piston valve; and
more preferably, a loading valve is arranged between said first mixing chamber and an upstream sample-receiving module (e.g. a sample loading chamber), which is a piston valve comprising a valve chamber and a valve piston disposed within the valve chamber.

4. The chip device according to any one of claims 1-3, **characterized in that** said sample lysis module of said sample lysis and nucleic acid extraction module comprises a lysis reagent storage chamber for holding the lysis reagent, which is a piston-style container comprising a chamber and a piston disposed within the chamber;
optionally, said sample lysis module further comprises a lysis reagent valve arranged between said lysis reagent storage chamber and a chamber for sample lysis, said lysis reagent valve being a piston valve;
preferably, said sample lysis and nucleic acid extraction module has one or more of a binding unit, a cleaning unit and an elution unit, which have a nucleic acid binding reagent storage chamber, a nucleic acid cleaning reagent storage chamber or a nucleic acid elution reagent storage chamber for holding a nucleic acid binding reagent, a nucleic acid cleaning reagent and a nucleic acid elution reagent, respectively, and each of said nucleic acid binding reagent storage chamber, nucleic acid cleaning reagent storage chamber and nucleic acid elution reagent storage chamber is a piston-style container comprising a chamber and a piston disposed within the chamber; and
optionally, said nucleic acid binding reagent storage chamber, nucleic acid cleaning reagent storage chamber and nucleic acid eluting reagent storage chamber are each connected to a chamber in which the nucleic acid binding, nucleic acid reagent or nucleic acid eluting reaction is carried out via a nucleic acid binding reagent valve, a nucleic acid cleaning reagent valve or a nucleic acid eluting reagent valve, respectively, and each of said nucleic acid binding reagent valve, nucleic acid cleaning reagent valve or nucleic acid eluting reagent valve is a piston valve.

5. The chip device according to claim 1, **characterized in that** said sample lysis and nucleic acid extraction module further comprises a sample-to-be-amplified chamber, which is a piston-style container comprising a chamber and a piston disposed in the chamber for holding a sample of the lysed and extracted nucleic acid solution; and
preferably, a piston valve is arranged upstream of the sample-to-be-amplified chamber, and comprises a valve chamber and a valve piston disposed within the valve chamber.

6. The chip device according to claim 1, **characterized in that** said sample lysis and nucleic acid extraction module further comprises a dilution chamber, preferably with a dilution valve arranged between said dilution chamber and its upstream module or unit (e.g., a second mixing chamber), which is a piston-style container.

7. The chip device according to claim 1, **characterized in that** said nucleic acid amplification module has one or more multiplexed amplification zone units, said multiplexed amplification zone units comprising a plurality of amplification reaction chambers for carrying out a nucleic acid amplification reaction, wherein the nucleic acid sample solution from the sample lysis and extraction module is distributed to each of said amplification reaction chambers by means of the microfluidic channel; and
optionally, a piston valve is arranged between said multiple amplification zone unit and an upstream sample lysis and extraction module (e.g., between said multiple amplification zone unit and an upstream sample-to-be-amplified chamber).

8. The chip device according to claim 7, **characterized in that** said plurality of amplification reaction chambers of said multiple amplification zone unit are distributed along a circumferential direction, optionally with a multiple amplification loading valve thereabove, which is a piston valve with a piston inside the valve chamber, which has an opening in communication with a flow channel connected to the upstream sample-to-be-amplified chamber at a bottom thereof, and with a plurality of openings connected to each of a plurality of amplification reaction chambers therebelow.

9. The chip device according to claim 7, **characterized in that** the plurality of amplification reaction chambers of said multiple amplification zone unit is arranged in an array, optionally with each amplification reaction chamber being connected to a main flow channel of said amplification zone unit via a branch flow channel, and the main flow channel of said multiple amplification zone unit being connected to an upstream sample lysis and nucleic acid extraction module.

10. The chip device according to claim 1, **characterized in that** said nucleic acid amplification module has an inert liquid module for storing and providing an inert liquid (e.g., mineral oil or paraffin oil) that covers an interface between the amplification reaction chamber and the flow path; and
for example, said inert liquid module has an oil filling chamber and, optionally, further comprises an oil filling valve between the oil filling chamber and the multiple amplification zone unit, which is a piston valve having a piston in the valve chamber body and an opening at a bottom of the valve chamber body thereof in communication with a flow path connected to the oil filling chamber, and an opening in communication with said amplification zone unit.

11. The chip device according to claim 1, **characterized in that** said nucleic acid amplification module further has a preamplification zone unit disposed upstream of said multiple amplification zone unit for carrying out a first round of amplification of nucleic acid molecules, e.g., for performing a nested amplification, and
that said preamplification zone unit has a nested amplification chamber, e.g., which is a cylindrical chamber disposed within a substrate having a chamber connected to an upstream sample lysis and nucleic acid extraction module and a downstream re-amplification zone unit, respectively, in connection with a flow channel.

12. The chip device according to any one of claims 1-11, **characterized in that** said piston valve has a piston movement control member provided above the valve piston, for controlling upward and downward movements of the valve piston within the chamber.

13. The chip device according to any one of claims 1-12, **characterized in that** the chamber wall of the valve chamber of said piston valve has an internal thread, said piston motion control member has an external thread to form a screw thread pair with the internal thread of said chamber wall, and the piston motion control member can rotate along the thread to move within the chamber for driving the piston to move in the chamber, and said piston motion control member has a cavity suitable for receiving a control rod, said cavity having a cross-section adapted to the control rod.

14. The chip device according to any one of claims 1-13, **characterized in that** the bottom of the valve piston of said piston valve has a piston support body, said piston support body being a projection located at the bottom of the piston, which is made of elastic material.

15. An instrument, preferably a POCT instrument, for detection of nucleic acids in a sample, comprising a chip device as claimed in any one of claims 1-14, wherein said chip device has a substrate and piston-style containers, said piston-style containers being in communication with each other through a microfluidic channel, wherein each piston-style container comprises a chamber and a piston disposed within the chamber, the chamber having an opening in communication with said microfluidic channel at a bottom thereof, and
said chip device further has a piston valve disposed between the containers for controlling fluidic communication between interconnected containers, said piston valve comprising a valve chamber and a valve piston disposed within the valve chamber, the valve chamber having two or more openings in communication with a flow channel at a bottom thereof, the valve piston being configured to move to the bottom of the chamber for covering said openings, blocking communication of the chamber with the microfluidic channel, thereby blocking fluidic communication between containers connected through said microfluidic channel, the flow channel being in communication with the openings at the bottom of the chamber from a position under the chamber via an upward channel;
wherein said chip device comprises:
a sample receiving module;
a sample lysis and nucleic acid extraction module, used to perform a lysis reaction and/or a nucleic acid extraction reaction on the sample to obtain purified nucleic acids from the sample, and comprising a sample lysis module and/or a nucleic acid extraction module, and optionally, said nucleic acid extraction module comprising a nucleic acid binding unit, a nucleic acid washing unit, and a nucleic acid elution unit; and
a nucleic acid amplification module, for distributing nucleic acid samples to a plurality of amplification zone containers and for amplifying and/or detecting nucleic acid molecules, the nucleic acid amplification module comprising a multiplexed amplification zone module and, optionally, a preamplification zone module.

16. The instrument according to claim 15, **characterized in that** the instrument further optionally comprises:
a chip device receiving and motion controlling system;
magnets and a system for controlling movement of the magnets;
a control mechanism for regulating movement of a piston motion control member in said piston valve, said control mechanism comprising a control rod and a control rod motion mechanism, the control rod being capable of moving up and down or rotating, wherein said control rod motion mechanism comprises a component, such as a motor, for controlling said control rod to move up and down or rotate;
a signal detection module for detecting nucleic acid amplification products, for example a fluorescence detection system;
a system for temperature control of the nucleic acid amplification region of said chip; and
a system for analyzing and/or outputting nucleic acid amplification results.

17. A method of detecting nucleic acids in a sample using the chip device of any one of claims 1-14 or the instrument of any one of claims 15-16, said method comprising steps of:
providing the chip device of any one of claims 1-14 or an instrument having said chip device, wherein said chip device has a substrate and piston-style containers, said piston-style containers being in communication with each other through a microfluidic channel, wherein each piston-style container comprises a chamber and a piston disposed within the chamber, the chamber having an opening in communication with said microfluidic channel at a bottom thereof, and
said chip device further has a piston valve disposed between the containers for controlling fluidic communication between interconnected containers, said piston valve comprising a valve chamber and a valve piston disposed within the valve chamber,
wherein said chip device comprises:
a sample receiving module;
a sample lysis and nucleic acid extraction module, used to perform a lysis reaction and/or a nucleic acid extraction reaction on the sample to obtain purified nucleic acids from the sample, and comprising a sample lysis module and/or a nucleic acid extraction module, and optionally, said nucleic acid extraction module comprising a nucleic acid binding unit, a nucleic acid washing unit, and a nucleic acid elution unit; and
a nucleic acid amplification module, for distributing nucleic acid samples to a plurality of amplification zone containers, e.g., amplification reaction chambers, and for amplifying and/or detecting nucleic acid molecules, the nucleic acid amplification module comprising a multiplexed amplification zone module and, optionally, a preamplification zone module.
wherein the instrument further optionally comprises:
a chip device receiving and motion controlling system;
magnets and a system for controlling movement of the magnets;
a control mechanism for regulating movement of a piston motion control member in said piston valve, said control mechanism comprising a control rod and a control rod motion mechanism, the control rod being capable of moving up and down or rotating, wherein said control rod motion mechanism comprises a component, such as a motor, for controlling said control rod to move up and down or rotate;
a signal detection module for detecting nucleic acid amplification products, for example a fluorescence detection system;
a system for temperature control of the nucleic acid amplification region of said chip; and
a system for analyzing and/or outputting nucleic acid amplification results,
wherein said method optionally comprises steps of:
adding a sample to be tested via a sample receiving module of said chip device;
performing a lysis reaction and/or a nucleic acid extraction reaction on the sample via the sample lysis and nucleic acid extraction module of said chip device to obtain purified nucleic acids from the sample;
dispensing the nucleic acid sample to a plurality of amplification zone containers, and amplifying and/or detecting nucleic acid molecules via said nucleic acid amplification module.
